# EUROPEAN PATENT SPECIFICATION

(11) **EP 2 670 359 B1**
(45) Date of publication and mention of the grant of the patent: **31.05.2017**
(21) Application number: 12700050.3
(22) Date of filing: 09.01.2012
(51) Int. Cl.: A61F 2/36, A61F 2/00, A61F 2/30

(54) **FEMORAL STEM FOR HIP PROSTHESIS**
FEMURSCHAFT FÜR HÜFTPROTHESE
TIGE FÉMORALE POUR PROTHÈSE DE HANCHE

(30) Priority: 01.02.2011 IT MI20110122
(43) Date of publication of application: 11.12.2013
(73) Proprietor: Adler Ortho S.R.L., 20032 Cormano (IT); Toni, Aldo, 40137 Bologna (IT)
(72) Inventor: TONI, Aldo, I-40137 Bologna (IT); CREMASCOLI, Patrizio, I-20091 Bresso (IT)
(74) Representative: Modiano, Micaela Nadia
(86) International application number: PCT/EP2012/050261
(87) International publication number: WO 2012/104115

(56) References cited:
- EP-A1- 0 011 665
- EP-A2- 0 864 303
- FR-A1- 2 837 695
- US-A1- 2006 167 557

## Description

The present invention relates to a femoral stem for a hip prosthesis. More particularly, the invention relates to a femoral stem that is used for hip prostheses in combination with a femoral neck with which a head and a cotyloid cavity are mated.

As is known, a hip prosthesis is constituted by a femoral stem which is inserted in the medullary canal of the femur and in which a neck is inserted which has a head that protrudes from the femoral stem and engages a cotyloid cavity, which in turn is accommodated in the provided seat of the pelvis, so as to reconstruct the hip joint. Document FR 2837695 discloses the features of the preamble of claim 1. Document US 2006/0167557 discloses a connection of a femoral neck to a head, wherein the male taper of the neck comprises ridges and grooves. One of the problems encountered most frequently with hip prostheses is due to the mating between the neck and the stem of the femur. This mating in fact can give rise to problems that can lead to breakage of the prosthesis. In particular, the mating between the neck, i.e., the "male" part of the prosthesis, and the stem, i.e., the "female" part of the prosthesis, is provided with the neck and stem made of the same material, for example titanium alloy.

The use of a same material has the purpose of avoiding electrolysis problems. The material currently used, titanium alloy, is the one that has the best performance in terms of strength. However, titanium mated with titanium, in the presence of micro-movements, produces "debris" that can lead to what are called cold welds. Such cold welds constitute the failure point for the mating between the stem and the neck.

In order to achieve a very stable mating it is therefore necessary to provide a very tight tolerance between the two components, the stem and the neck; it is necessary to produce the impact of the two components with a certain force in order to achieve an interlocking between the neck and the stem, and it is further necessary to produce an impact in the direction of the mating axis.

However, it is sufficient for the interlocking to be less than perfectly precise to pass from a perfect mating to a mating with contact between the two components in a relatively small area, with an extremely high load concentration that leads to breakage.

The aim of the present invention is to provide a femoral stem that allows a mating with the corresponding neck that is substantially free from breakage.

Within this aim, an object of the present invention is to provide a femoral stem that can be made of the same material as the neck while offering a high-strength mating.

Another object of the present invention is to provide a femoral stem which, when the corresponding neck is inserted therein, avoids an unwanted interlocking, i.e., an imperfect mating between the neck and the stem of the femur.

Another object of the present invention is to provide a femoral stem that is highly reliable, relatively simple to provide and has competitive costs.

This aim, as well as these and other objects that will become better apparent hereinafter, are achieved by a femoral stem, particularly for a hip prosthesis, provided with a recess for the mating of a neck, characterized in that said recess has a rough inner surface, said roughness being constituted by scoring lines adapted to allow optimum mating between the neck and the stem.

Further characteristics and advantages of the invention will become better apparent from the description of preferred but non-limiting embodiments of the femoral stem according to the present invention, illustrated by way of non-limiting example in the accompanying drawings, wherein:

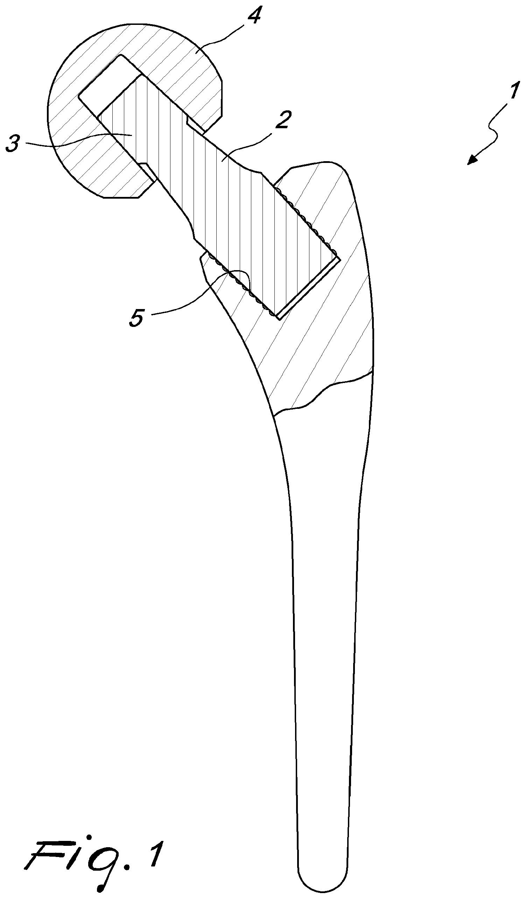

Figure 1 is a transverse sectional view of a femoral stem with a corresponding neck mated therewith, according to the present invention;
Figure 2a is a view of a first embodiment of the inner surface of the femoral stem according to the invention;
Figure 2b is a view of a second embodiment of the inner surface of the femoral stem according to the invention; and
Figure 2c is a view of a third embodiment of the inner surface of the femoral stem according to the invention.

With reference to the figures, the femoral stem according to the invention, generally designated by the reference numeral 1, is adapted to be mated with a femoral neck 2, which has, in its proximal part, what is called a "morse" cone 3, which is adapted to engage a femoral head 4, which in turn is articulated within a cotyloid cavity 6 which must be fixed in the adapted seat of the pelvis, in order to restore the hip joint.

The femoral stem 1 is provided with a channel or recess 5 within which the neck 2 that constitutes the male element of the mating is adapted to fit.

The femoral stem 1 and the neck 2 are preferably made of the same material, for example titanium alloy.

The peculiarity of the invention resides in that the channel or recess 5 defined within the femoral stem 1 has a surface with a greater roughness than what should be provided in order to have optimum contact surfaces.

When a kind of contact is provided, the intention is to obtain the lowest (smallest) possible roughness (measured in microns). This type of roughness (Rz) is the arithmetic mean of the five greatest peak-to-trough distances within the sampling length. The peaks and troughs are respectively the crests and hollows that define a rough surface.

Two components with a very low roughness Rz that mate have a very large contact surface. However, particularly if the two components are made of the same material, the possibility of a "jamming" (imperfect mating) is high. Therefore, the invention provides in the recess 5 such a roughness that the "peaks" that are created intentionally can be "deformed" by the neck 2 (male element of the mating), thus avoiding the above-mentioned unwanted jamming.

The roughness Rz that is provided in the recess 5 (measured in microns) is the distance between two parallel straight lines, one traced at the mean of the five highest peaks and the other one traced at the mean of the five lowest troughs, in the interval of the base length of the measurement.

It has been found that the roughness can be comprised between 6 and 50 microns, with a recommended optimum roughness between 20 and 30 microns.

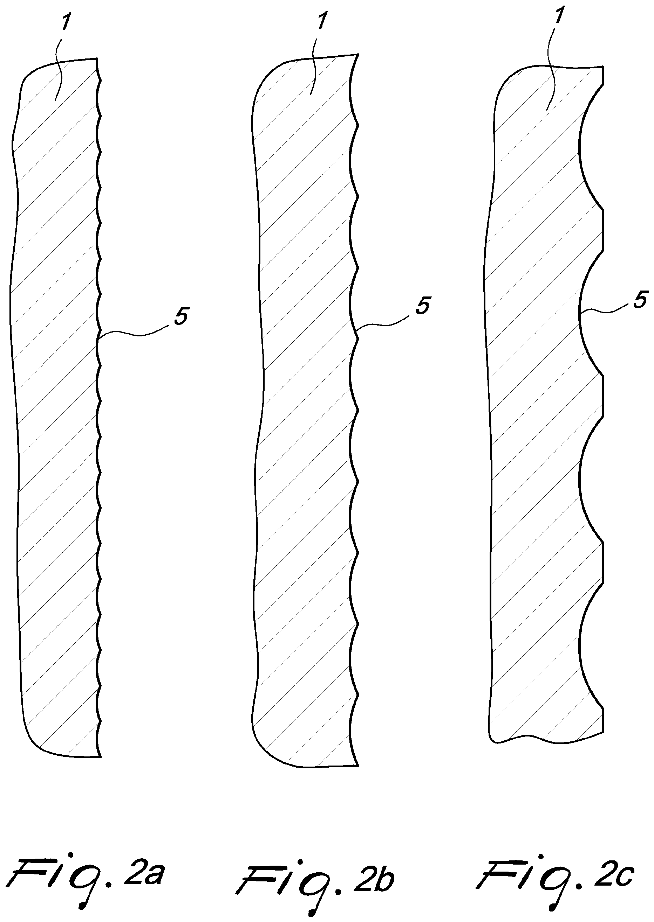

Figures 2a, 2b and 2c are views of three types of roughness, starting from 6 microns up to 50 microns of Figure 2c. The scoring lines that define the roughness have a pitch that can vary respectively between 75 microns for a 6-micron roughness and 350 microns for a 50-micron roughness.

The scoring lines described previously allow the male component (neck 2) to enter more easily the female component and, by bending the crests, thanks to their relative weakness, avoid the concentration of stresses and therefore the corresponding early breakages.

Conveniently, the scoring lines are arranged at right angles with respect to the axis of the mating between the stem and the distal part of the neck 2.

In practice it has been found that the femoral stem according to the invention fully achieves the intended aim and objects, since it allows, thanks to the scoring described on the inner surface of the recess, to provide a mating between the neck and the stem that makes it possible to enter the stem more easily, avoiding the possibility of early breakages caused by concentration of the stresses in specific points of the mating.

The stem thus conceived is susceptible of numerous modifications and variations, all of which are within the scope of the accompanying claims; all the details may further be replaced with other technically equipment elements.

In practice, the materials used, as well as the contingent shapes and dimensions, may be any according to requirements and to the state of the art.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A femoral stem (1), particularly for a hip prosthesis, provided with a recess (5) for the mating of a neck (2), said recess (5) having a rough inner surface, the roughness being constituted by scoring lines adapted to allow mating between the neck (2) and the stem (1), **characterized in that** said scoring lines are arranged at right angles to the axis of the mating between the stem and the distal part of the neck.

2. The femoral stem according to claim 1, **characterized in that** said roughness is comprised between 6 and 50 microns.

3. The femoral stem according to claim 1, **characterized in that** said roughness is comprised between 20 and 30 microns.

4. The femoral stem according to one or more of the preceding claims, **characterized in that** said scoring lines have a spacing comprised between 75 and 350 microns.

## Patentansprüche

1. Ein Femurschaft (1), insbesondere für eine Hüftprothese, ausgestattet mit einer Vertiefung (5) für das Einpassen eines Halses (2), wobei die Vertiefung (5) eine raue Innenfläche hat und die Rauheit aus Kerblinien besteht, ausgebindet, um eine Passung zwischen dem Hals (2) und dem Schaft (1) zu ermöglichten, **dadurch gekennzeichnet, dass** die Kerblinien in rechten Winkeln zur Achse der Passung zwischen dem Schaft und dem distalen Teil des Halses angeordnet sind.

2. Der Femurschaft gemäß Anspruch 1, **dadurch gekennzeichnet, dass** die Rauheit zwischen 6 und 50 Mikron beträgt.

3. Der Femurschaft gemäß Anspruch 1, **dadurch gekennzeichnet, dass** die Rauheit zwischen 20 und 30 Mikron beträgt.

4. Der Femurschaft gemäß einem oder mehreren der obigen Ansprüche, **dadurch gekennzeichnet, dass** die Kerblinien einen Abstand zwischen 75 und 350 Mikron haben.

## Revendications

1. Tige fémorale (1), en particulier pour une prothèse de hanche, munie d'une cavité (5) pour l'accouplement d'un col (2), ladite cavité (5) ayant une surface intérieure rugueuse, la rugosité étant constituée par des lignes d'entaille adaptées à accoupler le col (2) et la tige (1), **caractérisée en ce que** lesdites lignes d'entaille sont disposées à angle droit par rapport à l'axe de l'accouplement entre à tige et la partie distale du col,

2. Tige fémorale selon la revendication 1, **caractérisée en ce que** ladite rugosité est comprise entre 6 et 50 micromètres.

3. Tige fémorale selon la revendication 1, **caractérisée en ce que** ladite rugosité est comprise entre 20 et 30 micromètres.

4. Tige fémorale selon l'une ou plusieurs des revendications précédentes, **caractérisée en ce que** lesdites lignes d'entaille ont un espacement compris entre 75 et 350 micromètres.
